# FASCICULE DE BREVET EUROPEEN

(11) **EP 3 897 595 B1**
(45) Date de publication et mention de la délivrance du brevet: **15.03.2023**
(21) Numéro de dépôt: 19829158.5
(22) Date de dépôt: 19.12.2019
(51) Int. Cl.: A61K 31/05, A61K 31/07, A61K 31/202, A61K 31/355, A61K 31/375, A61K 33/30, A61K 33/34, A23L 33/00, A61P 27/02

(54) **COMPOSITION OPHTALMIQUE NUTRACEUTIQUE POUR LE TRAITEMENT DE PATHOLOGIES RETINIENNES A COMPOSANTE NEOVASCULAIRE**
OPHTHALMISCHE NUTRAZEUTISCHE ZUSAMMENSETZUNG FÜR DIE BEHANDLUNG VON RETINAERKRANKUNGEN MIT NEOVASKULÄRER KOMPONENTE
NUTRACEUTICAL OPHTHALMIC COMPOSITION FOR TREATING DISEASES OF THE RETINA WITH NEOVASCULAR COMPONENT

(30) Priorité: 20.12.2018 EP 18306772
(43) Date de publication de la demande: 27.10.2021
(73) Titulaire: Laboratoires THEA, 63100 Clermont-Ferrand (FR)
(72) Inventeur: OLMIERE, Céline, 63910 VERTAIZON (FR); MERCIER, Fabrice, 63400 CHAMALIERES (FR)
(74) Mandataire: Cabinet Laurent & Charras
(86) Numéro de dépôt international: PCT/EP2019/086174
(87) Numéro de publication internationale: WO 2020/127641

(56) Documents cités:
- WO-A1-2016/151269

## Description

### DOMAINE DE L'INVENTION

La présente invention concerne une composition ophtalmique nutraceutique comprenant notamment du resvératrol visant à être administrée chez un sujet recevant un traitement anti-VEGF, en particulier dans le cadre de la prise en charge d'une pathologie rétinienne à composante néovasculaire.

De manière surprenante, la présente demande démontre qu'une administration conjointe d'une telle composition et d'un agent anti-VEGF produit un effet bénéfique sur le taux de VEGF produit et offre des perspectives prometteuses pour le traitement de ces pathologies oculaires.

### ETAT DE LA TECHNIQUE

En Europe, la majorité des cas de cécité et de perte de vision sévère est due à des maladies de la rétine liées à l'âge. Compte-tenu des tendances démographiques actuelles, une augmentation de la prévalence et de l'incidence de ces maladies est attendue. Une des conséquences économiques directes sera une pression importante sur les systèmes de santé Européens.

A ce jour, la Dégénérescence Maculaire Liée à l'Age (DMLA) ou « Age-related Macular Degeneration » (AMD) affecte environ 34 millions de personnes en Europe. Le nombre de patients atteints de DMLA devrait augmenter de près de 25% au vu de la croissance démographique et du vieillissement de la population jusqu'en 2050. Par ailleurs et en ce qui concerne les pathologies oculaires liées au diabète, dont l'Œdème Maculaire Diabétique (OMD) ou « Diabetic Macular Oedema » (DMO), la situation actuelle et future est similaire. Plus de 25% des patients diabétiques sont touchés par une atteinte aux yeux (ou DED pour « Diabetic Eye Disease »), ce qui représente près de 4 millions d'individus en Europe. Comme la prévalence du diabète augmente considérablement en raison des tendances démographiques et des changements de mode de vie, ce type de pathologies augmentera également.

Plus précisément, la DMLA est la première cause de handicap visuel chez les personnes de plus de 50 ans. Toutes formes confondues, cette maladie concerne environ 8 % de la population française, mais sa fréquence augmente largement avec l'âge : elle touche 1 % des personnes de 50 à 55 ans, environ 10 % des 65-75 ans et de 25 à 30 % des plus de 75 ans. Si l'on tient compte uniquement des formes tardives de la maladie, associées à une perte de la vision centrale, ces chiffres sont à diviser environ par deux.

Cette pathologie touche sélectivement la région maculaire, c'est à dire la zone centrale de la rétine, entraînant une perte progressive de la vision centrale à savoir le champ de vision utile pour lire, reconnaître des visages ou conduire. Elle est la première cause de malvoyance chez les sujets âgés.

La maladie débute par une phase précoce, sans dégénérescence, appelée maculopathie liée à l'âge (MLA ou « sèche précoce »). Cette phase se caractérise par l'accumulation de petits dépôts blanchâtres (ou « drusen mous ») à l'intérieur et autour de la macula. Ces dépôts sont visibles lors d'un simple examen de fond d'oeil. Cette phase est le plus souvent asymptomatique, mais le patient peut éventuellement percevoir des déformations des lignes droites (« métamorphopsies ») et des taches floues.

Aux stades plus avancés, des symptômes apparaissent, variant en fonction de la gravité de la maladie:
- Diminution de l'acuité visuelle, avec nécessité d'avoir un meilleur éclairage pour la lecture ou pour tout travail de précision ;
- Vision centrale de plus en plus floue, altération de la perception des couleurs, distorsion des lignes droites, qui apparaissent déformées et gondolées ;
- Apparition d'une tache sombre au centre du champ visuel appelée scotome, difficultés à reconnaître les visages, hallucinations visuelles ou baisse brutale de l'acuité visuelle ;
- L'atteinte sévère du deuxième oeil est très variable d'un sujet à l'autre : elle peut survenir rapidement, dans un an, dans 10 ans, ou jamais.

Une MLA peut rester stable tout au long de la vie. Néanmoins, dans environ la moitié des cas et sous l'influence de plusieurs facteurs, la MLA évolue en formes dégénératives tardives :
- Forme atrophique qui est une forme sèche dans laquelle la macula s'atrophie en vieillissant et est progressivement remplacée par du tissu cicatriciel ; ou
- Forme humide qui est une forme exsudative dans laquelle de petits vaisseaux gorgés de sang se développent sous la macula. Saignant facilement, ces néovaisseaux sont responsables d'exsudats et d'hémorragies du fond d'oeil. Si une DMLA exsudative est présente à un oeil, il y a un risque de la développer au deuxième.

Ces deux formes tardives ont une incidence à peu près équivalente. Elles conduisent à une dégradation irréversible de la macula et à une perte de la vision centrale affectant un seul oeil ou les deux. Des formes mixtes peuvent être observées.

D'un point de vue mécanistique, la forme humide de la DMLA, dite néovasculaire ou exsudative, tout comme la rétinopathie diabétique, l'oedème maculaire, l'Occlusion Veineuse Rétinienne (OVR ou RVO) ou encore la forte myopie, se traduisent par une prolifération de nouveaux vaisseaux anormaux sous la rétine. Ces vaisseaux fragiles laissent diffuser du sérum, responsable d'un soulèvement de la rétine, et/ou du sang entraînant l'apparition d'hémorragies rétiniennes. Cette forme évolue rapidement si elle n'est pas prise en charge, avec une perte de la vision en quelques semaines ou même quelques jours. Ce processus peut être ralenti par la prise de médicaments de type anti-VEGF.

Le VEGF (pour « Vascular Endothelial Growth Factor ») est un facteur de croissance de l'endothélium vasculaire qui joue un rôle dans les processus d'angiogenèse, c'est-à-dire le bourgeonnement de vaisseaux préexistants, et de vasculogenèse correspondant à la formation de néo-vaisseaux à partir de progéniteurs endothéliaux. Le VEGF, et plus particulièrement l'isoforme VEGF-A, se fixe à la surface de récepteurs à activité tyrosine-kinase, notamment VEGF-R1 et VEGF-R2 qui jouent un rôle primordial dans l'angiogenèse.

Ainsi, à partir de 2006, certaines pathologies oculaires comme la forme humide de la DMLA (ou « AMD wet ») ont été traitées à l'aide d'inhibiteurs du VEGF. Progressivement, d'autres pathologies néovasculaires telles que l'oedème maculaire diabétique (OMD ou DME), l'occlusion veineuse rétinienne (OVR ou RVO) ou la néovascularisation choroïdienne (ou CNV) myopique (ou PM) ont également été traitées à l'aide de ces agents.

Parmi les agents anti-VEGF, on trouve notamment des anticorps ou fragments d'anticorps dirigés contre le VEGF ou encore des protéines recombinantes capables de se lier aux récepteurs du VEGF. A titre d'exemple, on peut citer le ranibizumab (par exemple le produit LUCENTIS^{®} commercialisé par NOVARTIS PHARMA SAS ou ses biosimilaires en développement par SAMSUNG, INTAS ou FORMYCON), le bevacizumab (par exemple le produit AVASTIN^{®} commercialisé par ROCHE), l'aflibercept (par exemple le produit EYLEA^{®} commercialisé par BAYER SANTE), l'abicipar pegol (en développement par ALLERGAN), le conbercept (par exemple le produit en développement par CHENGDU KANGHON BIOTECH INC), le faricimab (par exemple le produit RG7716 en développement par ROCHE), ou le Brolucizumab (NOVARTIS).

Ces agents anti-VEGF sont usuellement administrés par injection intravitréenne, à savoir une injection à travers la conjonctive et la sclérotique, directement dans la cavité oculaire située en arrière du cristallin, c'est-à-dire la cavité vitréenne contenant l'humeur vitrée.

Il s'agit donc de l'injection de substances médicamenteuses directement à l'intérieur de l'œil. Comparé à d'autres voies d'administration, notamment la voie topique, ce traitement comporte plusieurs avantages :
- une diminution de la quantité d'actif ;
- une action plus efficace ;
- un ciblage efficace de la zone à traiter ;
- une diminution des effets indésirables ou encore une réduction du passage systémique.

Toutefois, l'injection intravitréenne reste un geste technique, réalisé par des praticiens spécialisés et requérant des conditions particulières d'asepsie et d'antisepsie. Par ailleurs, cette injection peut être à l'origine d'un certain nombre d'effets secondaires assez problématiques: endophtalmie, détachement de la rétine, hypertension oculaire/glaucome, cataracte, inflammation, hémorragie subconjonctivale, toxicité systémique, inflammation, apparition de corps flottants,... En outre, il est généralement déconseillé de faire une injection dans les deux yeux le même jour afin d'éviter la perte de vision totale en cas de problème ou d'effet secondaires suite à l'injection. En effet, ces injections sont généralement suivies d'une période très douloureuse pour les patients qui peut les obliger à passer plusieurs heures dans le noir complet.

La posologie, en particulier la dose et la fréquence d'administration, peut notamment dépendre de l'actif mais également de la pathologie à traiter, avec une moyenne de sept injections par an.

A titre d'exemple, le traitement de la DMLA par le ranibizumab ou le bevacizumab commence par une phase d'induction avec 1 injection par mois pendant 3 mois consécutifs, suivie d'une phase de maintien au cours de laquelle l'acuité visuelle des patients est contrôlée une fois par mois. Si le patient présente une perte d'acuité visuelle de plus de 5 lettres à l'échelle « Early Treatment Diabetic Retinopathy Study » (ETDRS) ou l'équivalent d'une ligne sur l'échelle de Snellen, l'agent doit être à nouveau administré. L'intervalle entre deux doses ne doit pas être inférieur à 1 mois.

Pour la DME, des injections mensuelles de ranibizumab ou de bevacizumab sont préconisées jusqu'à stabilisation de l'acuité visuelle lors de trois évaluations mensuelles consécutives sous traitement.

Pour la RVO, des injections mensuelles de ranibizumab ou de bevacizumab sont préconisées jusqu'à stabilisation de l'acuité visuelle. L'absence d'amélioration de l'acuité visuelle lors de 3 injections consécutives mensuelles justifie l'arrêt du traitement.

### En présence d'aflibercept, le protocole est un peu différent :

Pour la DMLA, à l'instauration du traitement et comme pour les autres agents anti-VEGF, l'aflibercept est injecté 1 fois par mois pendant 3 mois consécutifs, puis une injection tous les 2 mois, sans visite de suivi entre les injections. Après les 12 premiers mois de traitement, l'intervalle entre 2 injections peut être prolongé en fonction des résultats visuels et anatomiques. Dans ce cas, le calendrier de suivi doit être déterminé par le médecin administrant le traitement et les visites de suivi peuvent être plus fréquentes que les injections programmées.

Pour l'OVR, la dose recommandée est de 2 mg d'aflibercept, correspondant à 50 µL. Après la première injection, le traitement est administré tous les mois. L'intervalle entre 2 injections ne doit pas être inférieur à 1 mois. Si aucune amélioration sur les paramètres visuels et anatomiques n'est observée à l'issue des trois premières injections, la poursuite du traitement n'est pas recommandée. Le traitement mensuel est poursuivi jusqu'à l'obtention de résultats visuels et anatomiques stables au cours de trois évaluations mensuelles. La nécessité de poursuivre le traitement peut ensuite être réévaluée. Si nécessaire, le traitement peut être poursuivi avec une augmentation progressive de l'intervalle entre 2 injections afin de maintenir la réponse visuelle et anatomique.

En parallèle de ces traitements à base d'agents anti-VEGF administrés par injection intravitréenne, d'autres techniques sont mises en oeuvre pour détruire les néo-vaisseaux, notamment la photocoagulation (destruction thermique des vaisseaux anormaux) et la photodynamique. Cette dernière technique consiste à injecter par voie intraveineuse un produit photosensible (par exemple vertéporfine) qui devient toxique sous l'effet de lumière rouge appliquée localement à l'aide d'un laser.

En amont de ces traitements lourds, mis en oeuvre à des stades avancés de la pathologie, des études ont été menées pour tenter de trouver des prises en charge plus précoces et plus « légères », ayant pour but de prévenir l'apparition et retarder l'avancée de la maladie.

Ainsi, des études appelées AREDS 1 (Age-Related Eye Disease Study Research Group. A Randomized, Placebo-Controlled, Clinical Trial of High-Dose Supplementation With Vitamins C and E, Beta Carotene, and Zinc for Age-Related Macular Degeneration and Vision Loss: AREDS Report No. 8 and 9. Arch Ophthalmol. 2001) et AREDS 2 (Lutein + zeaxanthin and omega-3 fatty acids for age-related macular degeneration: the Age-Related Eye Disease Study 2 (AREDS2) randomized clinical trial. JAMA. 2013 May 15;309(19):2005-15. doi: 10.1001/jama.2013.4997) ont été menées dans le but de démontrer l'effet bénéfique de compléments alimentaires sur les maladies oculaires.

Ces études ont révélé que des suppléments alimentaires à base d'antioxydants étaient potentiellement bénéfiques en relation avec des maladies oculaires, notamment celles liées à l'âge, en particulier la DMLA.

En pratique, l'étude AREDS 1 préconise la prise quotidienne d'antioxydants de type oligoéléments et vitamines, comme suit :

| | |
|---|---|
| Zinc | 80 mg |
| Vitamine C | 500 mg |
| Vitamine E | 267 mg |
| Cuivre | 2 mg |
| Béta-carotène (= Vitamine A) | 15 mg |

Ces supplémentations pourraient diminuer le risque de la perte de vision parmi les patients souffrant d'une DMLA avancée.

L'étude AREDS 2 a été menée pour mettre en évidence les effets de la lutéine, de la zéaxanthine (en remplacement du beta carotène) et de la supplémentation en acides gras oméga 3. Cette étude a préconisé la suppression de la vitamine A et rapporté le bénéfice de la prise quotidienne supplémentaire des éléments suivants:

| | |
|---|---|
| Lutéine | 10 mg |
| Zéaxanthine | 2 mg |
| acide docosahexaénoïque (DHA) | 350 mg |
| acide eicosapentaénoïque (EPA) | 650 mg |

Sur la base des résultats des études AREDS, il a donc été préconisé la prise quotidienne de tels compléments alimentaires chez les personnes présentant un risque élevé de développer une DMLA, en particulier chez les malades présentant une MLA à l'un ou aux deux yeux, ainsi que chez les patients atteints d'une DMLA avancé ne touchant encore qu'un oeil. Cependant, ces études n'ont pas permis de mettre en évidence un effet positif de ces formules sur la progression de la maladie chez des patients souffrant de DMLA avancée ni sur la perte de vision.

L'étude NAT 2 (Merle et al., Invest Ophthalmol Vis Sci. 2014 Mar 28;55(3):2010-9. doi: 10.1167/iovs.14-13916), incluant 300 patients sur trois ans, a permis de démontrer une réduction de 68% des risques de développement de DMLA néo-vasculaire après une augmentation significative du DHA dans les cellules. Ainsi, cette étude est la première explorant le potentiel d'une supplémentation orale en DHA sur une longue durée pour prévenir le développement de la DMLA.

Enfin, le document WO 2016/151269 décrit un complément alimentaire combinant des vitamines, des oligoéléments, des caroténoïdes, des acides gras oméga 3 et du resvératrol, et son utilisation pour le traitement et/ou la prévention de pathologies oculaires. En particulier, ce document rapporte que la combinaison des différents composés listés a une action différentielle et bénéfique sur l'expression et l'activité du VEGF ou de ses récepteurs (VEGF-R).

Il existe toutefois un besoin évident de développer de nouvelles solutions permettant d'améliorer la prise en charge des pathologies oculaires, notamment les pathologies rétiniennes à composante néovasculaire, répondant en particulier aux attentes suivantes : un espacement des injections intravitréennes d'anti-VEGF, une diminution des effets secondaires liées à celles-ci, une réduction des coûts de santé public et une amélioration de la qualité de vie des patients.

### EXPOSE DE L'INVENTION

De manière surprenante, le Demandeur a mis en évidence que l'administration d'un complément alimentaire permettrait d'augmenter, prolonger voire améliorer les effets des traitements anti-VEGF actuels, notamment des traitements par injection intravitréenne, chez des patients recevant de tels traitements.

Selon un premier aspect, l'invention concerne une composition ophtalmique nutraceutique comprenant du resvératrol et l'administration d'une telle composition à un sujet recevant un traitement anti-VEGF, en particulier soumis à un traitement par un agent anti-VEGF.

Dans le cadre de l'invention, on appelle « composition nutraceutique », une composition dont le but est de compléter le régime alimentaire normal et constituant une source concentrée de nutriments ou d'autres substances ayant un effet nutritionnel ou physiologique, seuls ou combinés. Il s'agit avantageusement d'un complément ou supplément alimentaire.

Dans le cadre de l'invention, on appelle « composition ophtalmique », une composition présentant des propriétés bénéfiques pour la santé oculaire. Ceci n'exclut toutefois pas la possibilité qu'une telle composition ait un effet bénéfique au niveau d'autres organes de l'homme ou de l'animal.

Le resvératrol est un composé polyphénolique, dérivé du stilbène dont il existe deux isomères mais la forme *trans* est majoritairement active. Dans la suite de la description, le terme « resvératrol » pourra donc être utilisé en lieu et place de trans-resvératrol.

Le resvératrol est un polyphénol retrouvé en quantités abondantes dans certains fruits, notamment dans le raisin et les oléagineux, et dans le vin. Il existe aujourd'hui plusieurs sources commerciales de resvératrol qui peuvent être utilisées pour la préparation d'une composition mise en oeuvre dans le cadre de l'invention :
- *Vitis vinifera :*
   ∘ Peau de raisin
   ∘ Pépin de raisin
- *Polygonum Cuspidatum*
- *Produit par fermentation*

Des sources privilégiées sont notamment :
- Resveratrol source *Vitis vinifera,* peau de raisin : par exemple un extrait naturel concentré à 5% ;
- Resveratrol synthétique 99% (CAS Number: 501-36-0).

Le resvératrol est avantageusement présent en quantité (dose journalière) supérieure ou égale à 1 mg, voire 2 mg, 5 mg, 10 mg, 20 mg, 30 mg, 50 mg, 60 mg voire même 100 mg ou 200 mg. Selon un mode de réalisation particulier, sa quantité journalière est supérieure ou égale à 20 mg voire supérieure à 20 mg, supérieure ou égale à 30 mg voire supérieure à 30 mg, voire supérieure ou égale à 50 mg, voire encore supérieure ou égale à 60 mg, voire même supérieure ou égale à 100 mg. Avantageusement, elle est inférieure ou égale à 1 g, de préférence inférieure ou égale à 500 mg, de manière particulièrement préférée inférieure ou égale à 200 mg. Selon un mode de réalisation particulier, une composition mise en oeuvre dans le cadre de l'invention comprend une dose journalière en resvératrol égale à 50 mg, 60 mg, 70mg, 80 mg, 90 mg, 100 mg, 150 mg ou 200 mg.

Selon un mode de réalisation avantageux, la composition comprend en outre des vitamines, des oligoéléments, des caroténoïdes, et des acides gras oméga 3. De manière encore plus avantageuse, les ingrédients vitamines, oligoéléments, acides gras oméga 3 et caroténoïdes, connus pour leurs propriétés antioxydantes, sont choisis comme préconisés par les études AREDS.

Selon un mode de réalisation particulier, la composition comprend au moins une vitamine, avantageusement une combinaison de vitamines. Il s'agit avantageusement de la vitamine C et/ou de la vitamine E.

Il peut également s'agir des vitamines suivantes :
- nicotinamide ou niacinamide ou vitamine B3 ou PP ;
- pyridoxine, par exemple sous forme de pyridoxine HCl, ou vitamine B6 ;
- riboflavine ou vitamine B2 ;
- thiamine, par exemple sous forme de thiamine nitrate, ou vitamine B1 ;
- cyanocobalamine ou vitamine B12 ;
- acide folique ou vitamine B9 ;
- Vitamine B5 ;
- Vitamine B8 ;
- Vitamine D.

La vitamine C, disponible sous forme d'acide ascorbique ou d'ascorbate de calcium, est avantageusement présente en quantité (dose journalière) comprise entre 50 et 500 mg, par exemple égale à 240 mg ou 120 mg.

La vitamine E ou alpha-tocophérol, par exemple disponible sous forme d'huile à 67%, est avantageusement présente en quantité (dose journalière) comprise entre 10 et 500 mg, par exemple égale à 30 mg.

Selon un autre mode de réalisation, des quantités quotidiennes suivantes, exprimées en mg, peuvent être envisagées pour les vitamines suivantes :
- vitamine B3 ou PP : 1 à 100, par exemple 18 ;
- vitamine B6 : 1 à 10, par exemple 2 ;
- vitamine B2 : 1 à 10, par exemple 1,6 ;
- vitamine B1 : 1 à 10, par exemple 1,4 ;
- vitamine B12 : 0,0005 à 0,01, par exemple 0,001 ;
- vitamine B9 : 0,1 à 1, par exemple 0,2.

Selon un mode de réalisation particulier, la composition ne comprend pas de vitamine A ou bêta-carotène.

Selon un mode de réalisation particulier, la composition comprend au moins un oligoélément, avantageusement une combinaison d'oligoéléments. Il s'agit avantageusement du zinc (Zn) et/ou du cuivre (Cu). Il peut également s'agir des oligoéléments suivants :
- du manganèse, par exemple du sulfate de manganèse anhydre ;
- du sélénium, par exemple du sélénite de sodium ;
- du magnésium, par exemple de l'oxyde de magnésium.

Le zinc (Zn), disponible sous forme d'oxyde de zinc ou de sulfate de zinc (monohydrate), est avantageusement présent en quantité (dose journalière) comprise entre 5 et 100 mg, par exemple égale à 12,5 mg.

Le cuivre (Cu), disponible sous forme de sulfate de cuivre, mono hydrate ou anhydre, est avantageusement présent en quantité (dose journalière) comprise entre 0,2 et 10 mg, par exemple égale à 1 mg.

Selon un autre mode de réalisation, des quantités quotidiennes suivantes, exprimées en mg, peuvent être envisagées pour les oligoéléments suivants :
- du manganèse : entre 1 et 10 mg, par exemple 1 mg ;
- du sélénium : entre 0,01 et 0,1 mg, par exemple 0,025 mg ;
- du magnésium : entre 1 et 50 mg, par exemple 10 mg.

Selon un mode de réalisation particulier, la composition comprend au moins un caroténoïde, avantageusement une combinaison de caroténoïdes. Il s'agit avantageusement de la lutéine et/ou de la zéaxanthine. Il peut également s'agir des caroténoïdes suivantes :
- de la méso-zéaxanthine ;
- du lycopène ;
- de l'astaxanthine.

La lutéine, disponible sous forme de lutéine à 20% en poids, est avantageusement présente en quantité (dose journalière) comprise entre 2 et 50 mg, par exemple égale à 10 mg ou 20 mg.

La zéaxanthine, disponible sous forme de zéaxanthine à 5 ou 14% en poids, est avantageusement présente en quantité (dose journalière) comprise entre 0,5 et 10 mg, par exemple égale à 2 mg ou 4 mg.

Selon un mode de réalisation particulier, la composition comprend au moins un acide gras polyinsaturé de type oméga 3, avantageusement une combinaison d'acides gras oméga 3. Il s'agit avantageusement de l'acide eicosapentaénoïque (EPA) et/ou de l'acide docosahexaénoïque (DHA). Il peut également s'agir des acides gras oméga 3 suivants :
- de l'acide alpha-linolénique (ALA) ;
- de l'acide docosapentaénoïque (DPA).

Les acides gras oméga 3 mis en oeuvre dans le cadre d'une telle composition peuvent être issus de l'huile de poisson riche en acides gras oméga 3, ou d'huile végétale, par exemple de lin. Ces acides gras sont avantageusement présents en quantité (dose journalière) comprise entre 100 et 1000 mg, Plus généralement, l'huile de poisson peut être présente en quantité journalière comprise entre 400 et 1000 mg, voire entre 800 et 1000 mg.

Dans ce contexte, l'EPA est avantageusement présent en quantité (dose journalière) comprise entre 100 et 1000 mg, par exemple égale à 172 mg.

Dans ce contexte, le DHA est avantageusement présent en quantité (dose journalière) comprise entre 100 et 1000 mg, par exemple égale à 366 mg.

Selon des modes de réalisation particulier, la composition mise en oeuvre dans le cadre de l'invention peut contenir d'autres actifs tels que :
- un ou d'autres polyphénol(s), tels que l'épigallocatechine-3-gallate (EGCG) ;
- du glutathion, par exemple à hauteur de 1 à 10 mg par jour, voire 2 mg par jour ;
- un ou des anthocyanoside(s) ;
- de l'hydroxythyrosol.

La teneur en chacun de ces ingrédients est aisément déterminée et ajustée par l'homme du métier.

Ainsi et selon une variante, la composition comprend ou est constituée des ingrédients suivants :
- vitamine C ;
- vitamine E ;
- éventuellement vitamine D ;
- zinc ;
- cuivre ;
- lutéine ;
- zéaxanthine ;
- acides gras oméga 3 issus de l'huile de poisson, notamment EPA et DHA ;
- resvératrol.

Selon une autre variante, la composition comprend ou est constituée des ingrédients suivants, sur la base d'une dose quotidienne:
- de 50 mg à 500 mg de vitamine C, avantageusement 240 mg, de manière particulièrement avantageuse 120 mg ;
- de 10 mg à 500 mg de vitamine E, avantageusement 30 mg ;
- de 5 mg à 100 mg de zinc, avantageusement 12,5 mg ;
- de 0,2 mg à 10 mg de cuivre, avantageusement 1 mg ;
- de 2 mg à 50 mg de lutéine, avantageusement 20 mg ;
- de 0,5 mg à 10 mg de zéaxanthine, avantageusement 4 mg ;
- de 400 mg à 1000 mg d'huile de poisson, de manière particulièrement avantageuse 800 à 1000 mg ;
- du resvératrol, avantageusement en quantité supérieure ou égale à 20 mg, de préférence supérieure à 30 mg, de manière particulièrement préférée supérieure à 50 mg, de manière très avantageuse en quantité supérieure ou égale à 60 mg ;
- éventuellement de la vitamine D, avantageusement 5 µg.

Selon un mode de réalisation préféré, la composition comprend ou est constituée des ingrédients suivants, sur la base d'une dose quotidienne:
- 120 mg de vitamine C;
- 30 mg de vitamine E;
- 12,5 mg de zinc ;
- 1 mg de cuivre;
- 20 mg de lutéine;
- 4 mg de zéaxanthine;
- 950 mg d'huile de poisson contenant avantageusement 366 mg de DHA et 172 mg de EPA ;
- du resvératrol, avantageusement en quantité supérieure ou égale à 50 mg, de manière très avantageuse en quantité supérieure ou égale à 60 mg.

Alternativement, la composition comprend ou est constituée de :
- 240 mg de vitamine C;
- 30 mg de vitamine E;
- 12,5 mg de zinc ;
- 1 mg de cuivre;
- 10 mg de lutéine;
- 2 mg de zéaxanthine;
- 950 mg d'huile de poisson ;
- du resvératrol, avantageusement en quantité supérieure ou égale à 20 mg, de manière très avantageuse en quantité supérieure ou égale à 30 mg.

Selon un mode de réalisation particulier, une telle composition est dépourvue d'au moins un des ingrédients de la liste suivante :
- le safran ;
- un agent chélateur des métaux, tels que l'EDTA ou l'acide phytique ;
- un nucléotide ;
- le pycnogénol ;
- un extrait de *ginkgo biloba.*

Selon ce mode de réalisation, la composition mise en oeuvre ne combine pas du resvératrol avec du safran, ou du resvératrol avec un agent chélateur des métaux et un ou plusieurs nucléotides, ou du resvératrol et du pycnogénol et/ou un extrait de *ginkgo biloba.*

Une composition mise en oeuvre dans le cadre de l'invention peut se présenter sous forme liquide, de solution, de suspension, de pâte ou de gel.

Avantageusement, elle se présente sous forme solide, telle que poudre, tablettes y compris tablettes effervescentes, capsules, gélules, comprimés ou pastilles, préparés de manière conventionnelle à l'aide d'additifs acceptables tels que des agents liants, des agents de comblement, des lubrifiants, des agents de désintégration ou des agents mouillants. Les tablettes peuvent optionnellement être « coatées » de manière connue de l'homme du métier à l'aide de sucres, de films ou de revêtements entériques.

De manière alternative, d'autres systèmes de délivrance peuvent être mis en oeuvre, tels que des préparations dans de la gélatine molle ou des capsules à base de gélatine ou des formulations préparées à l'aide des nanotechnologies, telles que des nano-dispersions, nano-émulsions ou nano-encapsulations.

Une telle composition peut en outre contenir un ou plusieurs additifs tels que colorants, pigments, arômes, ...

Selon un mode de réalisation particulier, la composition se présente sous forme de capsules, définissant un volume donné dans lequel est contenue la composition. La capsule se décompose dans l'appareil digestif afin de libérer les principes actifs et permettre son assimilation par l'organisme.

Dans ce contexte, des additifs classiques sont :
- du glycérol, par exemple du glycérol monostéarate ; et/ou
- de l'huile de soja ; et/ou
- de la cire d'abeille ; et/ou
- de la lécithine de soja.

De manière adaptée, ces additifs sont ajoutés à la composition en raison de leur capacité à améliorer par exemple les propriétés d'écoulement ou l'homogénéisation de celle-ci.

En outre et de manière connue de l'homme du métier, la tunique des capsules peut être élaborée à partir des ingrédients suivants :
- gélatine ; et/ou
- oxyde de fer rouge ; et/ou
- oxyde de fer noir ; et/ou
- sorbitol ; et/ou
- glycérol.

De manière connue de l'homme du métier, la répartition dans ce type de conditionnement relève d'un compromis entre :
- un nombre limité de prises, pour assurer une bonne observance du traitement ;
- un volume adapté des prises, permettant la formulation de l'ensemble des ingrédients et une ingestion aisée.

Avantageusement, une telle composition est administrée quotidiennement, à savoir de manière journalière.

Selon un mode de réalisation avantageux, cette composition est formulée dans 2 capsules, chacune contenant les ingrédients listés ci-dessus en quantité deux fois moindre.

Selon une première alternative, tous ces ingrédients sont mélangés et administrés dans une composition unique. Alternativement, ces ingrédients peuvent être combinés de différentes manières pour être formulés et/ou administrés de manière simultanée ou décalée dans le temps.

De manière caractéristique, la composition mise en oeuvre dans le cadre de l'invention est administrée par voie orale, avantageusement sous la forme de 2 prises quotidiennes. Selon un mode de réalisation particulier, la composition se présente sous forme de 2 capsules pouvant être prises avec un peu d'eau. Leur prise peut être simultanée, par exemple en début ou au cours du repas principal.

Au vu de l'effet mis en évidence dans les exemples, une telle composition est destinée à l'administration chez un sujet recevant un traitement anti-VEGF.

Selon un autre aspect, un traitement anti-VEGF tel qu'il sera détaillé ci-dessous est destiné à l'administration chez un sujet prenant une composition telle que définie ci-dessus, avantageusement par prise orale, encore plus avantageusement par prise orale quotidienne.

Selon un mode de réalisation particulier et dans le cadre de l'invention, un traitement anti-VEGF correspond à la prise d'un agent anti-VEGF, plus précisément un agent inhibiteur de la voie de signalisation du VEGF.

Avantageusement, l'agent anti-VEGF est choisi parmi un anticorps, un fragment d'anticorps, une protéine recombinante, une protéine de fusion et leur mélange.

Selon un mode de réalisation particulier, l'agent anti-VEGF est choisi dans le groupe constitué de : le ranibizumab, le bévacizumab, l'aflibercept, l'abicipar pegol, le conbercept, le faricimab, le brolucizumab, leurs biosimilaires et leurs mélanges.

Dans le cadre de l'invention, un biosimilaire s'entend au sens commun de ce terme, à savoir un médicament similaire à une substance biologique produite à partir d'une cellule ou d'un organisme vivant appelée médicament de référence. Ce médicament biosimilaire doit avoir des propriétés physico-chimiques et biologiques similaires, la même substance pharmaceutique et la même forme pharmaceutique que le médicament de référence, ainsi qu'une efficacité et une sécurité équivalentes.

Le ranibizumab est un fragment d'anticorps issu d'un anticorps monoclonal humanisé dirigé contre le VEGF-A.

Le bévacizumab est un anticorps monoclonal humanisé recombinant dirigé contre le VEGF.

L'aflibercept est une protéine de fusion recombinante correspondant aux domaines extracellulaires des récepteurs au VEGF de type 1 et 2 humains (VEGF-R1 et VEGF-R2) fusionnés au fragment cristallisable (Fc) d'une immunoglobuline IgG1 humaine.

L'abicipar pegol est une protéine conçue par répétition de l'ankyrine qui se lie à toutes les isoformes de VEGF-A. Sa petite taille (34 kDa) laisse supposer une demi-vie intra-oculaire courte, mais une pégylation, c'est-à-dire l'ajout de molécules de polyéthylène glycol lui permet d'acquérir des caractéristiques pharmacocinétiques d'une protéine plus grosse notamment une demi-vie plus longue.

Le conbercept est similaire à l'aflibercept en ce qu'il s'agit d'une protéine de fusion recombinante correspondant aux domaines extracellulaires clefs des récepteurs au VEGF de type 1 et 2 humains et de IgG Fc. Cependant, le conbercept contient aussi le quatrième domaine du récepteur VEGF 2.

Le faricimab est un anticorps monoclonal bispécifique anti-angiopoiétine 2 et anti-VEGF.

Le brolucizumab (ou RTH258, Novartis) est un anticorps mono-chaîne qui permet d'atteindre des concentrations en anticorps beaucoup plus élevées dans l'oeil que les traitements actuellement homologués. Cet anticorps a une très forte affinité pour toutes les isoformes du VEGF-A et devrait permettre d'espacer les injections intravitréennes.

De manière classique, ces agents anti-VEGF sont administrés par injection intravitréenne. Toutefois, au vu de la potentialisation de leur effet en présence d'une composition telle que décrite ci-dessus, leur administration par voie topique, avantageusement au niveau de la surface oculaire, peut être envisagée.

La combinaison de ces deux traitements (composition opthalmique nutraceutique par voie orale et traitement anti-VEGF par injection intravitréenne ou par voie topique) est avantageuse dans le cadre du traitement des pathologies oculaires, avantageusement des pathologies rétiniennes à composante néovasculaire, encore plus avantageusement choisies dans le groupe constitué de :
- la dégénérescence maculaire liée à l'âge, avantageusement la forme humide de la DMLA ;
- l'oedème maculaire diabétique (OMD);
- l'occlusion veineuse rétinienne (OVR);
- la néovascularisation choroïdienne myopique ;
- le glaucome néovasculaire ; et
- la rétinopathie diabétique proliférant.

Selon un autre aspect, la présente invention concerne un produit de combinaison ou une préparation combinée contenant la composition nutraceutique et le traitement anti-VEGF tels que décrits ci-dessus pour utilisation simultanée, séparée ou séquentielle dans le traitement d'une pathologie oculaire, avantageusement une pathologie rétinienne à composante néovasculaire, encore plus avantageusement choisie dans le groupe constitué de :
- la dégénérescence maculaire liée à l'âge, avantageusement la forme humide de la DMLA ;
- l'oedème maculaire diabétique (OMD) ;
- l'occlusion veineuse rétinienne (ORV);
- la néovascularisation choroïdienne myopique ;
- le glaucome néovasculaire ; et
- la rétinopathie diabétique proliférant.

Comme déjà dit et dans ce contexte, la composition nutraceutique est avantageusement administrée par voie orale, encore plus avantageusement de manière quotidienne, par exemple sous forme de 2 capsules prises au début ou au milieu du repas principal.

Le traitement anti-VEGF est, quant à lui, administré par injection intravitréenne ou voie topique. L'injection intravitréenne reste la voie d'administration privilégiée mais les développements en cours permettent d'envisager une administration par voie topique, avantageusement par application au niveau de la surface oculaire.

En effet et comme démontré, cette combinaison permet de réduire la posologie, notamment du traitement anti-VEGF. Ainsi, il est possible de diminuer les doses appliquées, ou avantageusement d'espacer les administrations (ou diminuer la fréquence d'administration) qui sont typiquement espacées de 1 à 3 mois.

En d'autres termes, ce produit de combinaison permet que la dose et/ou la fréquence du traitement anti-VEGF soit diminuée en comparaison avec la dose et/ou la fréquence appliquées à un sujet ne recevant pas la composition nutraceutique.

Selon un autre aspect, l'intervalle entre deux traitements anti-VEGF peut être supérieur de 30 jours, avantageusement supérieur de 45 jours, de préférence supérieur de 60 jours voire de 75 jours, plus avantageusement supérieur de 90 jours par rapport à la posologie actuelle.

Ainsi et selon un autre aspect, l'intervalle entre deux traitements anti-VEGF est supérieur à 30 jours, avantageusement supérieur à 45 jours, de préférence supérieur à 60 jours voire à 75 jours, plus avantageusement supérieur à 90 jours.

Il est ainsi possible de diminuer la potentielle toxicité des traitements anti-VEGF, notamment leurs effets secondaires. L'espacement de leur administration, qui requiert l'intervention de spécialistes, est également intéressant du point de vue économique.

### EXEMPLES DE REALISATION DE L'INVENTION

L'invention et les avantages qui en découlent ressortiront mieux des figures et exemples suivants donnés, afin d'illustrer l'invention et non de manière limitative.

### DESCRIPTION DES FIGURES

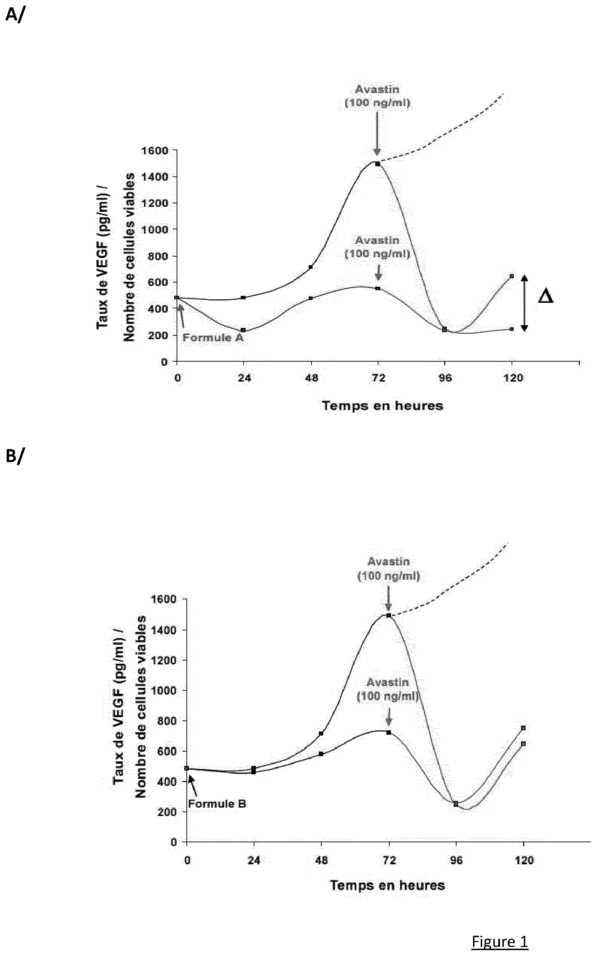

La figure 1 compare, dans des cellules rétiniennes indifférenciées (pathologiques) incubées (A) en présence (courbe inférieure) et en absence (courbe supérieure) de la formulation A, ou (B) en présence (courbe inférieure) et en absence (courbe supérieure) de la formulation B, l'évolution du taux de VEGF après administration de AVASTIN^{®}.

### A/ Protocole :

### Culture Cellulaire

*In vitro* les cellules indifférenciées rétiniennes humaines ARPE-19 (ATCC^{®} CRL-2302^{™}) sont cultivées dans du milieu DMEM (Dulbecco's modified eagle medium / nutrition mix F12 (1:1 Gibco, Paisley, UK) enrichi avec 10% de sérum de veau foetal (SVF, Dominique DUTSCHER, origine Amérique du Sud, lot n°S15197S1810), 1% de PSA (cocktail antibiotique : Pénicilline, Streptomycine, et Amphotéricine B respectivement à 10 000U/mL : 10 mg/mL : 25µg /mL, PAN Biotech) et 15mM d'HEPES (pH 7,4, Gibco). Les cellules sont cultivées dans une atmosphère à 37°C et 5% de CO₂. Ces cellules peuvent être assimilées à des cellules rétiniennes atteintes ou pathologiques.

### Traitements

Les cellules sont cultivées et ensemencées dans des boîtes de culture 24 puits à raison de 60 000 cellules par puits dans du milieu DMEM/F12 10% SVF et 1% PSA. 24 heures après leur ensemencement, le milieu de culture est éliminé au profit d'un milieu de culture restrictif DMEM/F12 avec 1% de SVF et 1% de PSA sans rouge de phénol. Après 24h supplémentaires, les cellules sont mises en présence ou non de la formulation A (avec resvératrol) ou de la formulation B (sans resvératrol).

La composition des formulations A et B est décrite ci-dessous :

**Tableau 1 : composition de la formulation A (avec resvératrol)**

| **Ingrédient** | **Quantités en mg/capsule** | **Concentrations en mg/g** | **Quantités en mg/jour** |
|---|---|---|---|
| Huile de poisson^{∗} dont | 475 | 688 | 950 |
| *DHA* | *183* | *265* | *366* |
| *EPA* | *86* | *125* | *172* |
| Vitamine C | 60 | 87 | 120 |
| Vitamine E | 15 | 22 | 30 |
| Zinc | 6.25 | 9.1 | 12.5 |
| Cuivre | 0.5 | 0.7 | 1 |
| Lutéine | 10 | 14.5 | 20 |
| Zeaxanthine | 2 | 2.9 | 4 |
| **Resveratrol** | **30** | **43** | **60** |
| *^{∗}: Huile Omegavie^{®} TG Quality Silver* | | | |

A noter qu'une capsule contient 690 mg de préparation et qu'il est préconisé une dose journalière de 2 capsules.

**Tableau 2 : composition de la formulation B (sans resvératrol)**

| **Ingrédients** | **Quantités en mg/capsule** | **Concentrations en mg/g** | **Quantités en mg/jour** |
|---|---|---|---|
| Huile de poisson^{∗} dont | 512.3 | 674 | 512.3 |
| *DHA* | *250* | *329* | *250* |
| *EPA (*≥*)* | *56* | *74* | *56* |
| *Vitamine B1* | *1.4* | *1.8* | *1.4* |
| *Vitamine B2* | *1.6* | *2.1* | *1.6* |
| *Vitamine B3* | *18* | *24* | *18* |
| *Vitamine B6* | *2* | *3* | *2* |
| *Vitamine B9* | *0.2* | *0.3* | *0.2* |
| *Vitamine B12* | *0.001* | *0.001* | *0.001* |
| Vitamine C | 60 | 79 | 60 |
| Vitamine E | 10 | 13 | 10 |
| Zinc | 7.5 | 9.9 | 7.5 |
| *Manganèse* | *1* | *1* | *1* |
| *Sélénium* | *0.025* | *0.03* | *0.025* |
| Cuivre | 1 | 1 | 1 |
| Lutéine | 10 | 13 | 10 |
| Zéaxanthine | 2 | 2.6 | 2 |
| *Glutathion* | *1* | *1* | *1* |

| | | | |
|---|---|---|---|
| *^{∗}: Huile TG* | | | |

A noter qu'une capsule contient 760 mg de préparation et qu'il est préconisé une dose journalière de 1 capsule.

Les formulations A et B sont reprises à la masse de 0.115g dans 1mL DMSO (DiMéthyl SulfOxyde), de sorte à obtenir une équivalence en resvératrol de 10 mM pour la formulation A et 0 mM pour la formulation B. Les concentrations en huile de poisson, vitamines C et E, Zinc, Cuivre Lutéine et Zéaxanthine sont considérées équivalentes dans les 2 préparations. A partir de cette solution, des dilutions sont effectuées directement dans le milieu de culture DMEM/F12 1% SVF, 1% PSA sans rouge de phénol pour atteindre des concentrations de 10 et 20 µM nécessaires à l'expérimentation.

Après 24h de pré-incubation, les cellules sont traitées avec de l'AVASTIN^{®} 100ng/mL (± formulation A ou B).

### ELISA (Enzyme-linked immunosorbent assay)

Au moment du traitement (t0) par l'Avastin^{®} 100ng/mL ainsi que 24 heures, 48 heures, et 72 heures après, le surnageant des cellules est prélevé afin d'analyser la quantité de VEGF-A relargué. Chaque ELISA est réalisé selon les instructions précises du fabricant (Kit ELISA VEGF-A Humain BMS277 affymetrix eBioscience).

### Cristal Violet

Le tapis cellulaire est analysé par cristal violet afin de déterminer la viabilité cellulaire. Un ratio entre le taux de VEGF-A provenant du surnageant et le tapis cellulaire correspondant peut être effectué.

Le tapis cellulaire est délicatement rincé 2 fois avec du tampon PBS 1X. Lorsque l'excédent de liquide est éliminé, le tapis cellulaire est fixé à son support par de l'éthanol absolu. Après une incubation d'au moins 30 minutes, l'éthanol absolu est éliminé. Les cellules sont mises en contact avec la solution de coloration au cristal violet pendant 20 minutes à température ambiante et sous oscillation (25 oscillations par minutes).

La solution de cristal violet est éliminée, et les puits sont rincés 4 fois avec de l'eau afin d'éliminer l'excédent de coloration. Les plaques sont ensuite séchées à température ambiante (pendant 16 à 24 heures). La coloration cellulaire est ensuite reprise en suspension dans une solution d'acide acétique 33%, les plaques sont mises sous oscillation puis la densité optique peut être lue par spectrométrie à la longueur d'onde de 570 nm (OD₅₇₀).

### B/ Résultats :

On peut observer que la présence de A permet de limiter la quantité de VEGF produite mais aussi de retarder de façon significative la remontée de ce taux après traitement avec un anti-VEGF, ce qui permettrait d'espacer les injections d'anti-VEGF.

La formulation B, quant à elle, entraine une diminution de la quantité de VEGF produite mais n'entraine pas de délai dans la reprise de la production de VEFG dans les cellules rétiniennes traitées avec l'anti-VEGF.

Cette différence inattendue est liée à la présence de resvératrol à haute concentration dans la formulation A.

## Revendications

1. Composition ophtalmique nutraceutique comprenant du resvératrol, avantageusement en quantité supérieure à 20 mg, pour utilisation dans le traitement d'une pathologie oculaire chez un sujet recevant un traitement anti-VEGF.

2. Composition pour son utilisation selon la revendication 1, ***caractérisée* en ce que** la composition comprend une quantité supérieure ou égale à 30 mg de resvératrol, avantageusement supérieure à 30 mg, encore plus avantageusement supérieure ou égale à 50 mg.

3. Composition pour son utilisation selon l'une des revendications précédentes, ***caractérisée* en ce que** la composition comprend en outre:
- des vitamines, avantageusement choisies dans le groupe constitué par la vitamine C et la vitamine E ;
- des oligoéléments, avantageusement choisis dans le groupe constitué par le zinc et le cuivre ;
- des caroténoïdes, avantageusement choisis dans le groupe constitué par la zéaxanthine et la lutéine ;
- des acides gras oméga 3, provenant avantageusement de l'huile de poisson, encore plus avantageusement choisis dans le groupe constitué par l'acide eicosapentaénoïque (EPA) et l'acide docosahexaénoïque (DHA).

4. Composition pour son utilisation selon l'une des revendications précédentes, caractérisée en ce qua la composition comprend :
- de la vitamine C, avantageusement de 50 mg à 500 mg, encore plus avantageusement 120 mg ;
- de la vitamine E, avantageusement de 10 mg à 500 mg, encore plus avantageusement 30 mg ;
- du zinc, avantageusement de 5 mg à 100 mg, encore plus avantageusement 12,5 mg ;
- du cuivre, avantageusement de 0,2 mg à 10 mg, encore plus avantageusement 1 mg ;
- de la lutéine, avantageusement de 2 mg à 50 mg, encore plus avantageusement 20 mg ;
- de la zéaxanthine, avantageusement de 0,5 mg à 10 mg, encore plus avantageusement 4 mg ;
- des acides gras oméga 3 issus de l'huile de poisson, avantageusement de 100 mg à 1000 mg, encore plus avantageusement de 800 mg à 1000 mg.

5. Composition pour son utilisation selon l'une des revendications précédentes, ***caractérisée* en ce que** la composition est administrée par voie orale.

6. Composition pour son utilisation selon l'une des revendications précédentes, ***caractérisée* en ce que** la composition est administrée de manière quotidienne.

7. Composition pour son utilisation selon l'une des revendications précédentes, ***caractérisée* en ce que** le traitement anti-VEGF correspond à l'administration d'un inhibiteur de la voie de signalisation du VEGF, avantageusement choisi dans le groupe suivant : un anticorps, un fragment d'anticorps, une protéine recombinante, une protéine de fusion et leur mélange.

8. Composition pour son utilisation selon l'une des revendications précédentes, ***caractérisée* en ce que** l'inhibiteur est choisi dans le groupe suivant : le ranibizumab, le bévacizumab, l'aflibercept, l'abicipar pegol, le conbercept, le faricimab le brolucizumab et leurs mélanges.

9. Composition pour son utilisation selon l'une des revendications précédentes, ***caractérisée* en ce que** le traitement anti-VEGF est administré par injection intravitréenne ou par voie topique.

10. Composition pour son utilisation selon l'une des revendications précédentes, ***caractérisée* en ce que** la pathologie oculaire est choisie dans le groupe constitué de la dégénérescence maculaire liée à l'âge, l'oedème maculaire diabétique, l'occlusion veineuse rétinienne, la néovascularisation choroïdienne myopique, le glaucome néovasculaire et la rétinopathie diabétique proliférant.

11. Composition telle que définie dans l'une des revendications 1 à 4 pour utilisation simultanée, séparée ou séquentielle avec un agent anti-VEGF, avantageusement tel que défini dans les revendications 7 et 8, dans le traitement d'une pathologie oculaire, avantageusement choisie dans le groupe constitué de la dégénérescence maculaire liée à l'âge, l'oedème maculaire diabétique, l'occlusion veineuse rétinienne, la néovascularisation choroïdienne myopique, le glaucome néovasculaire et la rétinopathie diabétique proliférant.

12. Composition pour son utilisation selon la revendication 11, ***caractérisée* en ce que** la composition est administrée par voie orale, avantageusement de manière quotidienne.

13. Composition pour son utilisation selon la revendication 11 ou 12, ***caractérisée* en ce que** l'agent anti-VEGF est administré par injection intravitréenne ou voie topique.

14. Composition pour son utilisation selon l'une des revendications 11 à 13, ***caractérisée* en ce que** la dose et/ou la fréquence d'administration de l'agent anti-VEGF est diminuée en comparaison avec la dose et/ou la fréquence appliquées à un sujet ne recevant pas la composition.

15. Composition pour son utilisation selon la revendication 14, ***caractérisée* en ce que** l'intervalle entre deux administrations de l'agent anti-VEGF est supérieur de 30 jours, avantageusement supérieur de 45 jours, de préférence supérieur de 60 jours voire de 75 jours, plus avantageusement supérieur de 90 jours, par rapport à l'intervalle entre deux administrations de l'agent anti-VEGF chez un sujet ne recevant pas la composition.

## Patentansprüche

1. Ophthalmische nutrazeutische Zusammensetzung, umfassend Resveratrol, vorteilhafterweise in einer Menge von mehr als 20 mg, zur Verwendung bei der Behandlung einer Augenerkrankung bei einem Subjekt, das eine Anti-VEGF-Behandlung erhält.

2. Zusammensetzung zur Verwendung gemäß Anspruch 1, **dadurch gekennzeichnet, dass** die Zusammensetzung eine Menge von mehr als oder gleich 30 mg Resveratrol, vorteilhafterweise von mehr als 30 mg, noch vorteilhafter von mehr als oder gleich 50 mg umfasst.

3. Zusammensetzung zur Verwendung gemäß einem der vorhergehenden Ansprüche, **dadurch gekennzeichnet, dass** die Zusammensetzung ferner Folgendes umfasst:
- Vitamine, vorteilhafterweise ausgewählt aus der Gruppe bestehend aus Vitamin C und Vitamin E;
- Spurenelemente, vorteilhafterweise ausgewählt aus der Gruppe bestehend aus Zink und Kupfer;
- Carotinoide, vorteilhafterweise ausgewählt aus der Gruppe bestehend aus Zeaxanthin und Lutein;
- Omega-3-Fettsäuren, vorteilhafterweise aus Fischöl stammend, noch vorteilhafter ausgewählt aus der Gruppe bestehend aus Eicosapentaensäure (EPA) und Docosahexaensäure (DHA).

4. Zusammensetzung zur Verwendung gemäß einem der vorhergehenden Ansprüche, **dadurch gekennzeichnet, dass** die Zusammensetzung Folgendes umfasst:
- Vitamin C, vorteilhafterweise 50 mg bis 500 mg, noch vorteilhafter 120 mg;
- Vitamin E, vorteilhafterweise 10 mg bis 500 mg, noch vorteilhafter 30 mg;
- Zink, vorteilhafterweise 5 mg bis 100 mg, noch vorteilhafter 12,5 mg;
- Kupfer, vorteilhafterweise 0,2 mg bis 10 mg, noch vorteilhafter 1 mg;
- Lutein, vorteilhafterweise 2 mg bis 50 mg, noch vorteilhafter 20 mg;
- Zeaxanthin, vorteilhafterweise 0,5 mg bis 10 mg, noch vorteilhafter 4 mg;
- Omega-3-Fettsäuren aus Fischöl, vorteilhafterweise 100 mg bis 1000 mg, noch vorteilhafter 800 mg bis 1000 mg.

5. Zusammensetzung zur Verwendung gemäß einem der vorhergehenden Ansprüche, **dadurch gekennzeichnet, dass** die Zusammensetzung oral verabreicht wird.

6. Zusammensetzung zur Verwendung gemäß einem der vorhergehenden Ansprüche, **dadurch gekennzeichnet, dass** die Zusammensetzung täglich verabreicht wird.

7. Zusammensetzung zur Verwendung gemäß einem der vorhergehenden Ansprüche, **dadurch gekennzeichnet, dass** die Anti-VEGF-Behandlung der Verabreichung eines Inhibitors des VEGF-Signalwegs entspricht, der vorteilhafterweise ausgewählt ist aus der folgenden Gruppe: ein Antikörper, ein Antikörperfragment, ein rekombinantes Protein, ein Fusionsprotein und Mischungen davon.

8. Zusammensetzung zur Verwendung gemäß einem der vorhergehenden Ansprüche, **dadurch gekennzeichnet, dass** der Inhibitor aus der folgenden Gruppe ausgewählt ist: Ranibizumab, Bevacizumab, Aflibercept, Abicipar Pegol, Conbercept, Faricimab, Brolucizumab und Mischungen davon.

9. Zusammensetzung zur Verwendung gemäß einem der vorhergehenden Ansprüche, **dadurch gekennzeichnet, dass** die Anti-VEGF-Behandlung durch intravitreale Injektion oder topisch verabreicht wird.

10. Zusammensetzung zur Verwendung gemäß einem der vorhergehenden Ansprüche, **dadurch gekennzeichnet, dass** die Augenerkrankung ausgewählt ist aus der Gruppe bestehend aus altersbedingter Makuladegeneration, diabetischem Makulaödem, Netzhautvenenverschluss, myoper choroidaler Neovaskularisation, neovaskulärem Glaukom und proliferativer diabetischer Retinopathie.

11. Zusammensetzung, wie in einem der Ansprüche 1 bis 4 definiert, zur gleichzeitigen, getrennten oder aufeinanderfolgenden Verwendung mit einem Anti-VEGF-Mittel, vorteilhafterweise wie in den Ansprüchen 7 und 8 definiert, bei der Behandlung einer Augenerkrankung, die vorteilhafterweise ausgewählt ist aus der Gruppe bestehend aus altersbedingter Makuladegeneration, diabetischem Makulaödem, Netzhautvenenverschluss, myoper choroidaler Neovaskularisation, neovaskulärem Glaukom und proliferativer diabetischer Retinopathie.

12. Zusammensetzung zur Verwendung gemäß Anspruch 11, **dadurch gekennzeichnet, dass** die Zusammensetzung oral, vorteilhafterweise täglich, verabreicht wird.

13. Zusammensetzung zur Verwendung gemäß Anspruch 11 oder 12, **dadurch gekennzeichnet, dass** das Anti-VEGF-Mittel durch intravitreale Injektion oder topisch verabreicht wird.

14. Zusammensetzung zur Verwendung gemäß einem der Ansprüche 11 bis 13, **dadurch gekennzeichnet, dass** die Dosis und/oder die Häufigkeit der Verabreichung des Anti-VEGF-Mittels im Vergleich zu der Dosis und/oder der Häufigkeit verringert ist, die bei einem Subjekt angewandt wird, das die Zusammensetzung nicht erhält.

15. Zusammensetzung zur Verwendung gemäß Anspruch 14, **dadurch gekennzeichnet, dass** das Intervall zwischen zwei Verabreichungen des Anti-VEGF-Mittels mehr als 30 Tage, vorteilhafterweise mehr als 45 Tage, vorzugsweise mehr als 60 Tage oder sogar mehr als 75 Tage, noch vorteilhafter mehr als 90 Tage beträgt, verglichen mit dem Intervall zwischen zwei Verabreichungen des Anti-VEGF-Mittels bei einem Subjekt, das die Zusammensetzung nicht erhält.

## Claims

1. An ophthalmic nutraceutical composition comprising resveratrol, advantageously in an amount greater than 20 mg, for use in the treatment of an eye disease in a subject receiving anti-VEGF treatment.

2. The composition for its use according to claim 1, ***characterised* in that** the composition comprises an amount greater than or equal to 30 mg of resveratrol, advantageously greater than 30 mg, even more advantageously greater than or equal to 50 mg.

3. The composition for its use according to one of the preceding claims, ***characterised* in that** the composition further comprises:
- vitamins, advantageously selected from the group consisting of vitamin C and vitamin E;
- trace elements, advantageously selected from the group consisting of zinc and copper;
- carotenoids, advantageously selected from the group consisting of zeaxanthin and lutein;
- omega-3 fatty acids, advantageously from fish oil, even more advantageously selected from the group consisting of eicosapentaenoic acid (EPA) and docosahexaenoic acid (DHA).

4. The composition for its use according to any one of the preceding claims, ***characterised* in that** the composition comprises:
- vitamin C, advantageously from 50 mg to 500 mg, even more advantageously 120 mg;
- vitamin E, advantageously from 10 mg to 500 mg, even more advantageously 30 mg
- zinc, advantageously from 5 mg to 100 mg, even more advantageously 12.5 mg;
- copper, advantageously from 0.2 mg to 10 mg, even more advantageously 1 mg;
- lutein, advantageously from 2 mg to 50 mg, even more advantageously 20 mg;
- zeaxanthin, advantageously from 0.5 mg to 10 mg, even more advantageously 4 mg;
- omega-3 fatty acids from fish oil, advantageously from 100 mg to 1000 mg, even more advantageously from 800 mg to 1000 mg.

5. A composition for its use according to one of the preceding claims, ***characterised* in that** the composition is administered orally.

6. The composition for its use according to one of the preceding claims, ***characterised* in that** the composition is administered on a daily basis.

7. The composition for its use according to one of the preceding claims, ***characterised* in that** the anti-VEGF treatment corresponds to the administration of an inhibitor of the VEGF signalling pathway, advantageously chosen from the following group: an antibody, an antibody fragment, a recombinant protein, a fusion protein and mixtures thereof.

8. The composition for its use according to one of the preceding claims, ***characterised* in that** the inhibitor is selected from the following group: ranibizumab, bevacizumab, aflibercept, abicipar pegol, conbercept, faricimab, brolucizumab and mixtures thereof.

9. The composition for its use according to one of the preceding claims, ***characterised* in that** the anti-VEGF treatment is administered by intravitreal injection or topically.

10. The composition for its use according to one of the preceding claims, ***characterised* in that** the eye disease is selected from the group consisting of age-related macular degeneration, diabetic macular oedema, retinal vein occlusion, myopic choroidal neovascularisation, neovascular glaucoma and proliferative diabetic retinopathy.

11. The composition as defined in any of claims 1 to 4 for simultaneous, separate or sequential use with an anti-VEGF agent, advantageously as defined in claims 7 and 8, in the treatment of an eye disease, advantageously selected from the group consisting of age-related macular degeneration, diabetic macular oedema, retinal vein occlusion, myopic choroidal neovascularisation, neovascular glaucoma and proliferative diabetic retinopathy.

12. The composition for its use according to claim 11, ***characterised* in that** the composition is administered orally, advantageously on a daily basis.

13. The composition for its use according to either claim 11 or 12, ***characterised* in that** the anti-VEGF agent is administered by intravitreal injection or topically.

14. The composition for its use according to any one of claims 11 to 13, ***characterised* in that** the dose and/or frequency of the administration of the anti-VEGF agent is reduced in comparison with the dose and/or frequency applied to a subject who is not receiving the composition.

15. The composition for its use according to claim 14, ***characterised* in that** the interval between two administrations of the anti-VEGF agent is greater than 30 days, advantageously greater than 45 days, preferably greater than 60 days or even greater than 75 days, more advantageously greater than 90 days, compared with the interval between two administrations of the anti-VEGF agent in a subject not receiving the composition.
